# EUROPÄISCHE PATENTSCHRIFT

(11) **EP 0 382 056 B1**
(45) Veröffentlichungstag und Bekanntmachung des Hinweises auf die Patenterteilung: **20.07.1994**
(21) Anmeldenummer: 90101792.1
(22) Anmeldetag: 30.01.1990
(51) Int. Cl.: C07D 487/04

(54) **Verfahren zur Herstellung von cyclischen Amidinen**
Process for the preparation of cyclic amidines
Procédé de préparation d'amidines cycliques

(30) Priorität: 04.02.1989 DE 3903368
(43) Veröffentlichungstag der Anmeldung: 16.08.1990
(73) Patentinhaber: BASF Aktiengesellschaft, 67063 Ludwigshafen (DE)
(72) Erfinder: Hesse, Michael, Dr., D-6700 Ludwigshafen (DE); Scharf, Emil, Dr., D-6700 Ludwigshafen (DE)

(56) Entgegenhaltungen:
- EP-A- 0 158 319
- FR-A- 1 491 791
- GB-A- 1 182 014
- US-A- 3 761 436

## Beschreibung

Die vorliegende Erfindung betrifft ein Verfahren zur Herstellung von cyclischen Amidinen durch Umsetzung von Lactamen mit Diaminen in Gegenwart von Zeolithen als Katalysatoren.

Es ist bekannt, daß cyclische Amidine, die im allgemeinen starke organische Basen sind, ausgehend von Lactamen hergestellt werden können (Synthesis 1972 (11) 591 -598). Dies geschieht jedoch meist mehrstufig oder unter Verwendung von sehr giftigen und schwer handhabbaren Substanzen (Ang. Chemie, 79 (1967) 1, S. 53; JACS 103 (1981) 14, 4186 -94).

Nach Chem. Abstr. 74; 141 730c ist zum Beispiel Diazabicycloundecen (DBU) durch Umsetzung von Caprolactam mit Acrylnitril an einem basischen Katalysator, gefolgt von einer Hydrierung des so hergestellten Cyanoethylcaprolactams unter einem Druck bis 200 bar und einer anschließenden Kondensation zugänglich.

Aus der US-A-3 761 436 ist die Cyclisierung von N-Aminoalkyl-lactamen an Zinn- oder Antimonoxiden zu den entsprechenden Amidinen bekannt.

Die bei den oben genannten Verfahren z. T. notwendigen starken Säuren bei der Cyclisierung oder Basen bei der Cyanoethylierung werfen starke Korrosionsprobleme auf und müssen zusätzlich neutralisiert werden. Zudem sind alle bekannten Verfahren nur in mehreren Verfahrensschritten durchführbar.

Der vorliegenden Erfindung lag daher die Aufgabe zugrunde, ein Verfahren zur Herstellung von cyclischen Amidinen zu finden und den genannten Nachteilen abzuhelfen.

Demgemäß wurde neues und verbessertes Verfahren zur Herstellung von cyclischen Amidinen der allgemeinen Formel I 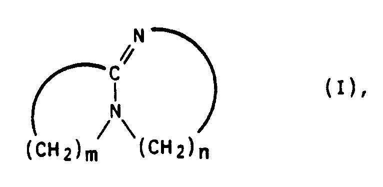
in der m und n für die Zahlen 2, 3, 4 oder 5 stehen, gefunden, welches dadurch gekennzeichnet ist, daß man Lactame der allgemeinen Formel II 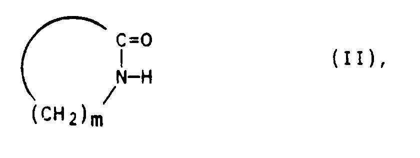
in der m für 2, 3, 4 oder 5 steht, mit Diaminen der allgemeinen Formel III

H₂N - (CH₂)ₙ - NH₂ (III),

in der n für 2, 3, 4 oder 5 steht, in Gegenwart von Zeolithen bei Temperaturen von 50 bis 500°C und Drücken von 0,01 bis 50 bar umsetzt.

Die cyclischen Amidine I sind nach folgender Methode erhältlich:
Die Umsetzung zu den cyclischen Amidinen I erfolgt durch gemeinsamen Kontakt von Lactamen II und Diaminen III an Zeolithen bei Temperaturen von 50 bis 500°C und Drücken von 0,01 bis 50 bar nach folgender Reaktionsgleichung: 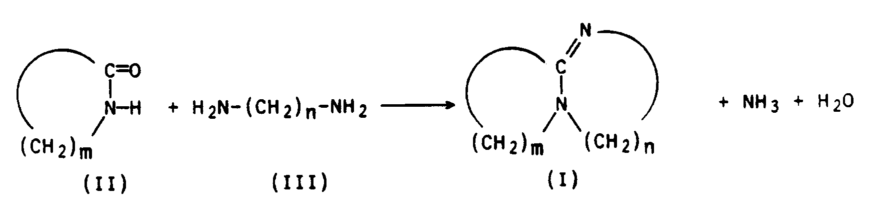
Die Reaktion kann sowohl in der Flüssigphase als auch diskontinuierlich oder vorzugsweise kontinuierlich in der Gasphase bei 50 bis 500°C und 0,01 bis 50 bar durchgeführt werden.

Die Flüssigphasenreaktion kann beispielsweise als Suspensions-, Riesel- oder Sumpfreaktion bei Temperaturen von 50 bis 200°C und Drücken von 0,5 bis 20 bar, vorzugsweise bei Temperaturen von 70 bis 170°C und Drücken von 1 bis 5 bar durchgeführt werden.

Die bevorzugte Gasphasenreaktion kann beispielsweise bei Temperaturen von 100 bis 500°C, vorzugsweise bei 150 bis 450°C und Drücken von 0,1 bis 50 bar, besonders bevorzugt bei 200 bis 400°C und Drücken von 0,5 bis

5 bar durchgeführt werden. Bei der Umsetzung in der Gasphase hält man vorteilhaft eine Katalysatorbelastung von 0,1 bis 20, insbesondere von 1 bis 10 g der Ausgangsstoffe der Formel II und III je g Katalysator und Stunde ein (WHSV = g Einsatzgemisch/g Katalysator und Stunde). Die Gasphasenreaktion kann in einem Festbett oder in einem Wirbelbett ausgeführt werden.

Es empfiehlt sich schwerflüchtige oder feste Ausgangsstoffe in einem inerten Lösungsmittel gelöst zu verwenden, wobei pro Mol der Ausgangsstoffe zwischen 100 und 500 ml, vorzugsweise 150 bis 350 ml eines inerten Lösungsmittels in aller Regel genügen.

Als inerte Lösungsmittel eignen sich Ether, besonders cyclische Ether wie Tetrahydrofuran; aliphatische Kohlenwasserstoffe wie n-Pentan, das Pentanisomerengemisch, n-Hexan, das Hexanisomerengemisch, Petrolether und Cyclohexan; aromatische Kohlenwasserstoffe wie Benzol, Toluol, die Xylole und deren Isomerengemisch oder Mischungen dieser Lösungsmittel.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bevorzugt werden gasförmigen Reaktionsprodukte sofort in eine Trennung eingebracht und z.B. in einer Fraktionierkolonne in ihre Einzelkomponenten zerlegt.

Das Molverhältnis der Einsatzstoffe Diamin zu Lactam liegt in der Regel zwischen 0,1:1 und 10:1, vorzugsweise zwischen 0,5:1 und 3:1.

Bevorzugte cyclische Amidine I sind:
Diazabicyclononen und
Diazabicycloundecen
Bevorzugte Lactame II sind:
2-Pyrrolidon
2-Piperidon
ε-Caprolactam
Bevorzugte Diamine (1,ω) III sind:
1,2-Diaminoethan
1,3-Diaminopropan
1,4-Diaminobutan
1,5-Diaminopentan
1,6-Diaminohexan
Als Katalysatoren für das erfindungsgemäße Verfahren verwendet man Zeolithe, zweckmäßig in der aciden Form. Zeolithe sind kristalline Aluminiumsilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Eikn Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, LO, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe.

In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus SiO₄-Tetraedern aufgebauten Fünfring gemeinsam.

Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisend. Es handelt sich hierbei um Aluminium-, Bor-, Eisen-, Beryllium-, Gallium-, Chrom-, Arsen-, Antimon- und Wismutsilikatzeolithe oder deren Gemische sowie Aluminium-, Bor-, Gallium- und Eisengermanatzeolithe oder deren Gemische. Insbesondere eignen sich die Aluminium-, Bor- und Eisensilikatzeolithe des Pentasiltyps für das erfindungsgemäße Verfahren. Der Aluminiumsilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit doder insbesondere ohne Alkali- oder Erdalkalizusatz bei 100 bis 220°C unter autogenem Druck hergestellt. Hierzu gehören auch die isotaktischen Zeolithe nach EP-A-34 727 und EP-A-46 504. Die erhaltenen Aluminiumsilikatzeolithe enthalten je nach Wahl der Einsatzstoffmengen ein SiO₂/Al₂O₃-Verhältnis von 10 bis 40 000. Aluminiumsilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen (SiO₂/Al₂O₃ ≧ 10) gehören auch die verschiedenen ZSM-Typen, Ferrierit, Nu-1 und Silicalit®.

Borsilikatzeolithe kann man z.B. bei 90 bis 200°C unter autogenem Druck synthetisieren, indes man eine Borverbindung, z.B. H₃BO₃, mit einer Siliciumverbindung, vorzugsweise hochdispersem Siliciumdioxid in wäßriger Aminlösung, insbesondere in 1,6-Hexandiamdin- oder 1,3-Propandiamin- oder Triethylentetrasin-Lösung mit und insbesondere ohne Alkali- oder Erdalkalizusatz zur Reaktion bringt. Hierzu gehdören auch die isotaktischen Zeolithe nach EP-A-34 727 und EP-A-46 504. Solche Borsilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeodlith erhält man z.B. aus einer Eisenverbindung, vorzugsweise Fe₂(SO₄)₃ und einer Siliciumverbindung, vorzugsweise hochdispersem siliciumdioxid in wäßriger Aminlösung, insbesondere 1,6-Hexandiamin, mit oder ohne Alkali- oder Erdalkalizusatz bei 100 bis 200°C unter autogenem Druck.

Die so hergestellten Aluminium-, Bor- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.-% zu Strängen oder Tabletten verformt werden. Als Bindemittel eignen sich diverse Aluminiumoxide, bevorzugt Boehmit, amorphe Aluminiumsilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Aluminium- bzw. Borsilikatzeolith direkt nach der Trocknung verformt wird und erst nach der Verformung einer Calcinierung unterworfen wird. Die hergestellten Aluminium- und Borsilikatzeolithe können in reiner Form ohne Binder als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden. Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Ba, Metalle der 3., 4. und 5. Hauptgruppe wie B, Al, Ga, Ge,d Sn, Pb, Bi, Seltene Erdmetalle wie La, Ce, Pr, Nd, Er, Yb und U eingesetzt. Die Modifizierung mit diesen Metallen kann durch Ionenaustausch oder Imprägnierung erfolgen.

Zweckmäßigerweise führt man diese Dotierung so durch, daß man den verformten Zeolithen in einem Steigrohr vorlegt und bei 20 bis 100°C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine weitere Möglichkeit der Modifizierung besteht darin, daß man das zeolithische Material - verformt oder unverformt - einer Behandlung mit Säuren wie Salzsäure, Flußsäure dund Phosphorsäure und/oder Wasserdampf unterwirft.

Im einzelnen geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 h getrocknet und bei 500°C/20 h calciniert. Nach einer anderen Arbeitsweise behandelt man Zeolithe vor oder nach ihrer Verformung mit Bindemittel, z.B. 1 bis 2 h bei Temperaturen von 60 bis 80°C mit einer 3 bis 25Gew.-%igen, insbesondere 12 bis 20 Gew.-%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n, Flußsäure eingesetzt wird, behandelt, beispielsweise durch Erhitzen unter Rückfluß über einen Zeitraum von im allgemeinen 0,5 bis 5, vorzugsweise 1 bis 2 h. Nach Isolierung, z.B. durch Abfiltrieren und Auswaschen des zeolithischen Materials, wird dieses zweckmäßig, z.B. bei Temperaturen von 100 bis 160°C, getrocknet und bei Temperturen von im allgemeinen 450°C bis 600°C calciniert. Gemäß einer weiteren bevorzugten Ausführungsform für die Säurebehandlung, wird das zeolithische Material nach einer Verformung mit Bindemittel bei erhöhter Temaperatur, zweckmäßig bei Temperaturen von 50 bis 90°C, vorzugsweise 60 bis 80°C, über einen Zeitraum von 0,5 bis 5 h, vorzugsweise mit 12 bis 20 Gew.-%iger Salzsäure, behandelt. Zweckmäßig wird das zeolithische Material anschließend ausgewaschen, bei Temperaturen von 100 bis 160°C getrocknet und bei Temperaturen von 450 bis 600°C calciniert. Einer HF-Behandlung kann sich auch eine HCl-Behandlung anschließen.

Wenn bei der erfindungsgemäßen Verwendung der zeolithischen Katalysatoren wie auch der anderen hierfür eingesetzten Katalysatoren evtl. eine durch Koksabscheidung bedingte Desaktivierung eintritt, empfiehlt es sich, diese durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4-mm-Stränge oder als Tabletten dmit 3 bis 5 mm Durchmesser oder als Splitt mit Teilchengrößen von 0,1 bis 0,5 mm oder als Wirbelkontakt eingesetzt werden.

### Herstellung der Katalysatoren

### Katalysator A

Katalysator A ist ein kommerziell erhältlicher Y-Zeolith in der H-Form.

### Katalysator B

Katalysator B ist ein kommerziell erhältlicher Y-Zeolith in der H-Form mit. einem Verhältnis von SiO₂/Al₂O₃ > 8 (US-Y).

### Katalysator C

Katalysator C wird aus Katalysator A hergestellt, durch Tränkung mit einer wäßrigen Lösung aus Ce-Nitrat. Der Ce-Gehalt beträgt 7,1 Gew.-%.

### Katalysator D

Der Borsilikatzeolith des Pentasil-Typs wird in einer hydrothermalen Synthese aus 740 g hochdispersem SiO₂, 360 g H₃BO₃, 9,7 kg einer wäßrigen 1,6-Diaminohexan-Lösung (50 Massen-% Amin) bei 165°C unter autogenem Druck in eines Rührautoklaven hergestellt. Nach Abfiltrieren und Auswaschen wird das kristalline Reaktionsprodukt bei 160°C 24 h getrocknet und bei 500°C 24 h calciniert. Dieser Borsilikatzeolith setzt sich zusammen aus 92,9 Gew.-% SiO₂ und 2,92 Gew.-% B₂O₃.

Der Borsilikatzeolith wird mit 0,08 n HF (3,6 g HF-Lösung/g Zeolith) 1 h unter Rückfluß gekocht. Danach wird der Zeolith abfiltriert, gewaschen, bei 110°C getrocknet und bei 500°C 5 h calciniert.

Der behandelte Borsilikatzeolith wird mit einem amorphen Aluminiumsilikat (Massenverhältnis SiO₂:Al₂O₃ = 75:25) im Massenverhältnis 60:40 unter Zusatz eines Verformungshilfsmittels zu 2-mm-Strängen verformt, die bei 110°C/16 h getrocknet und bei 500°C 16 h calciniert werden. Der F-Gehalt des Katalysators beträgt 0,055 Massen-%.

### Katalysator E

Ein Borsilikatzeolith des Pentasil-Typs wird in einer hydrothermalen Synthese aus 640 g hochdispersem SiO₂, 122 g H₃BO₃, 8000 g einer wäßrigen 1,6-Hexandiamin-Lösung (Mischung 50:50 Gew.-%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borsilikatzeolith setzt sich zusammen aus 94,2 Gew.-% SiO₂ und 2,3 Gew.-% B₂O₃. Daraus werden durch Verformen mit Boehmit im Gewichtsverhältnis 60:40 2-mm-Stränge hergestellt, die bei 110°C/16 h getrocknet und bei 500°C/24 h calciniert werden.

### Katalysator F

Katalysator F ist kommerziell erhältliches Kieselgel in Strangform.

### Katalysator G

Katalysator G ist kommerziell erhältliches Aluminiumoxid in Strangform.

### Beispiele

### Beispiele 1 bis 12

Als Ausgangssubstanzen werden epsilon-Caprolactam und 1,3-Diaminopropan im molaren Verhältnis von 1:1,5 eingesetzt, die WHSV beträgt ca. 3 h⁻¹.

Die Reaktion wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Tabellen I bis III geben die Umsätze bezogen auf eingesetztes Lactam und Selektivitäten zum gewünschten Amidin wieder.

### Beispiele 13 bis 21

Die Versuche werden wie die Beispiele 1 bis 12 durchgeführt mit der Ausnahme, daß als Ausgangssubstanzen epsilon-Caprolactam und 1,3-Diaminopropan im molaren Verhältnis von 1:2 eingesetzt werden.

### Beispiele 22 bis 25

Die Versuche werden wie die Beispiele 1 bis 12 durchgeführt mit der Ausnahme, daß als Ausgangssubstanzen 2-Pyrrolidon und 1,3-Diaminopropan im molaren Verhältnis von 1:1,5 eingesetzt werden.

**Tabelle I**

| Beispiel | Katalysator | Temperatur (°C) | Umsatz (%) | Selektivität (%) |
|---|---|---|---|---|
| 1 | A | 300 | 57 | 29 |
| 2 | A | 320 | 71 | 54 |
| 3 | A | 350 | 76 | 53 |
| 4 | A | 250 | 31 | 1 |
| 5 | B | 320 | 51 | 5 |
| 6 | B | 350 | 57 | 19 |
| 7 | B | 380 | 66 | 34 |
| 8 | C | 330 | 44 | 52 |
| 9 | F | 400 | 59 | 22 |
| 10 | F | 350 | 52 | 5 |
| 11 | F | 350 | 55 | 30 |
| 12 | E | 380 | 54 | 17 |

**Tabelle II**

| Beispiel | Katalysator | Temperatur (°C) | Umsatz (%) | Selektivität (%) |
|---|---|---|---|---|
| 13 | A | 300 | 52 | 19 |
| 14 | A | 350 | 97 | 23 |
| 15 | A | 400 | 57 | 5 |
| 16 | B | 350 | 93 | 28 |
| 17 | B | 400 | 98 | 12 |
| 18 | D | 350 | 54 | 19 |
| 19 | D | 400 | 49 | 18 |
| 20 | F | 350 | 59 | 10 |
| 21 | G | 350 | 96 | 7 |

**Tabelle III**

| Beispiel | Katalysator | Temperatur (°C) | Umsatz (%) | Selektivität (%) |
|---|---|---|---|---|
| 22 | A | 350 | 29 | 74 |
| 23 | A | 380 | 30 | 59 |
| 24 | B | 350 | 44 | 91 |
| 25 | B | 380 | 54 | 77 |

## Patentansprüche

1. Verfahren zur Herstellung von cyclischen Amidinen der allgemeinen Formel I 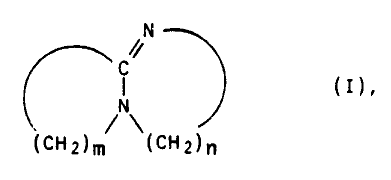 in der m und n für die Zahlen 2, 3, 4 oder 5 stehen, dadurch gekennzeichnet, daß man Lactame der allgemeinen Formel II 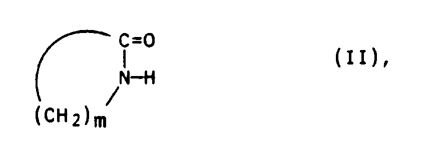 in der m für 2, 3, 4 oder 5 steht, mit Diaminen der allgemeinen Formel III
H₂N - (CH₂)ₙ - NH₂ (III),
in der n für 2, 3, 4 oder 5 steht, in Gegenwart von Zeolithen bei Temperaturen von 50 bis 500°C und Drücken von 0,01 bis 50 bar umsetzt.

2. Verfahren nach Anspruch 1, dadurch gekennzeichnet, daß man Diamin III zu Lactam II im Molverhältnis 0,1:1 bis 10:1 einsetzt.

3. Verfahren nach Anspruch 1, dadurch gekennzeichnet, daß der Index n für die Zahl 2 oder 3 steht.

4. Verfahren nach Anspruch 1, dadurch gekennzeichnet, daß man die Umsetzung in der Gasphase bei 100 bis 500°C und 0,01 bis 50 bar durchführt.

5. Verfahren nach Anspruch 1, dadurch gekennzeichnet, daß man als Katalysatoren Aluminiumsilikatzeolithe des Faujasit- , des Erionit- oder des Offretittyps verwendet.

6. Verfahren nach Anspruch 1, dadurch gekennzeichnet, daß man als Katalysatoren Aluminiumsilikatzeolithe, Borsilikatzeolithe oder Eisensilikatzeolithe des Pentasiltyps verwendet.

7. Verfahren nach Anspruch 1, dadurch gekennzeichnet, daß man Zeolithkatalysatoren verwendet, die mit einem übergangsmetall und/oder Edelmetall und/oder mit Seltenen Erden dotiert sind.

## Claims

1. A process for preparing cyclic amidines of the general formula I 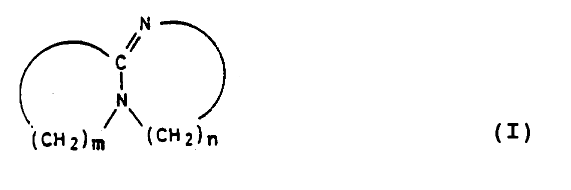 where m and n are each 2, 3, 4 or 5, which comprises reacting lactams of the general formula II 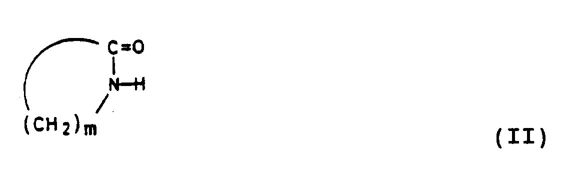 where m is 2, 3, 4 or 5, with diamines of the general formula III
H₂N - (CH₂)ₙ - NH₂ (III)
where n is 2, 3, 4 or 5, in the presence of zeolites at from 50 to 500°C and from 0.01 to 50 bar.

2. A process as claimed in claim 1, wherein diamine III is used relative to lactam II in a molar ratio from 0.1:1 to 10:1.

3. A process as claimed in claim 1, wherein the index n is 2 or 3.

4. A process as claimed in claim 1, wherein the reaction is carried out in the gas phase at from 100 to 500°C and from 0.01 to 50 bar.

5. A process as claimed in claim 1, wherein the catalyst used is an aluminosilicate zeolite of the faujasite, erionite or offretite type.

6. A process as claimed in claim 1, wherein the catalyst used is an aluminosilicate zeolite, a borosilicate zeolite or an iron silicate zeolite of the pentasil type.

7. A process as claimed in claim 1, wherein the zeolite catalyst used has been doped with a transition metal, a noble metal or a rare earth.

## Revendications

1. Procédé de préparation d'amidines cycliques de formule générale I 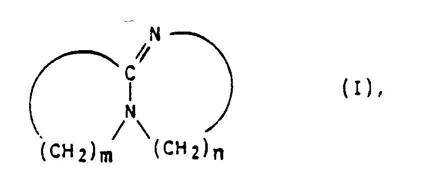 dans laquelle m et n sont mis pour les nombres 2, 3, 4 ou 5, caractérisé en ce qu'on fait réagir des lactames de formule générale II 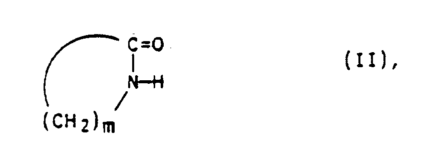 dans laquelle m est mis pour 2, 3, 4 ou 5, avec des diamines de formule générale III
H₂N - (CH₂)ₙ - NH₂ (III)
dans laquelle n est mis pour 2, 3, 4 ou 5, en présence de zéolithes à des températures de 50 a 500°C et des pressions de 0,01 à 50 bar.

2. Procédé selon la revendication 1, caractérisé en ce qu'on utilise la diamine III dans un rapport molaire au lactame II de 0,1:1 à 10:1.

3. Procédé selon la revendication 1, caractérisé en ce que l'indice n est mis pour le nombre 2 ou 3.

4. Procédé selon la revendication 1, caractérisé en ce que l'on effectue la réaction en phase gazeuse à une température de 100 à 500°C et une pression de 0,01 à 50 bar.

5. Procédé selon la revendication 1, caractérisé en ce qu'on utilise comme catalyseur des zéolithes d'aluminosilicate du type faujasite, érionite ou offrétite.

6. Procédé selon la revendication 1, caractérisé en ce qu'on utilise comme catalyseur des zéolithes d'aluminosilicate, des zéolithes de borosilicate ou des zéolithes de ferrosilicate du type pentasil.

7. Procédé selon la revendication 1, caractérisé en ce qu'on utilise des catalyseurs de zéolithe qui ont été dopés avec un métal de transition et/ou un métal noble et/ou un métal des terres rares.
